# DEMANDE DE BREVET EUROPEEN

(11) **EP 1 181 909 A1**
(43) Date de publication de la demande: **27.02.2002**
(21) Numéro de dépôt: 00202926.2
(22) Date de dépôt: 21.08.2000
(51) Int. Cl.: A61F 2/34

(54) **Armature d'ancrage acétabulaire élastique pour insert articulaire cotyloidien de hanche en matériau rigide**

(71) Demandeur: Bone & Joint Research S.A., 3644 Kayl (LU)
(72) Inventeur: Autrique, Jean-Claude, 1300 Wavre (BE); Boucquey, Pierre, 7700 Mouscron (BE); Deneufbourg, Jean, 1300 Wavre (BE); Geulette, Bernard, 1050 Bruxelles (BE); Ghosez, Jean-Pierre, 5081 Bovese (La Bruyere) (BE); Moulin, Jean, 3644 Kayl (LU); Thiery, Jacques, 1390 Grez-Doiceau (BE); Vander Maren, Christian, 1340 Ottignies (BE); Ghijselings, Ignace, B/9910 Ursel (LU); Manche, Eric, 1325 Corroy-le-Grand (BE); Labrique, Jean-Francois, 7711 Dottignies (BE)
(74) Mandataire: Schmitz, Jean-Marie

(57) **Abrégé**

Armature d'ancrage acétabulaire (1) élastique pour pièce ou insert articulaire cotyloidien (9) de hanche en matériau rigide à couple de frottement métal/métal ou matière céramique/matière céramique présentant au moins une zone déformable (2) dont l'épaisseur est volontairement réduite afin de permettre sa rétraction sans venir en contact avec l'insert ou pièce articulaire cotyloidien rigide (9), cette zone déformable (2) dépassant de la géométrie de révolution extérieure (3) de ladite armature (1) et se rétractant lors de la mise en place dans la cavité osseuse acétabulaire (11) par impaction en force avec un ajustage serré de manière à créer un effet ressort grâce à l'élasticité du matériau qui la constitue mettant en pression l'os dans la zone des cornes inférieures (12 et 13) pour s'opposer lors de la marche aux déformations d'ovalisation et de torsion de la cavité acétabulaire (11).

## Description

La présente invention concerne une armature d'ancrage acétabulaire élastique pour pièce articulaire cotyloïdienne de hanche selon la partie précaractérisante de la revendication 1.

Plus précisément, l'invention concerne une armature d'ancrage acétabulaire élastique pour pièce articulaire cotyloïdienne de hanche en matériau rigide à couple de frottement métal/métal ou céramique/céramique présentant au moins une zone déformable dépassant de la géométrie de révolution extérieure de ladite armature et se rétractant lors de la mise en place dans la cavité osseuse acétabulaire par impaction en force de préférence avec un ajustage serré, de manière à créer un effet ressort, grâce à l'élasticité du matériau de préférence métallique, mettant en pression l'os de préférence dans la zone des cornes inférieures pour s'opposer, lors de la marche, aux déformations d'ovalisation et de torsion de ladite cavité acétabulaire tendant à expulser les armatures rigides d'ancrage.

Il est connu d'utiliser des implants cotyloïdiens sans ciment dits impactés qui sont rentrés en force dans une cavité osseuse généralement sous dimensionnée par rapport à l'implant de manière à créer un effet d'arc-boutement assurant la tenue initiale pendant la période d'intégration osseuse.

Le FR-A-2 589 059 décrit un composant-cotyloïdien pour prothèse de hanche non cimenté qui comporte une coupole métallique hémisphérique et une cupule en matériau polymère qui s'y emboîte pour former garniture de friction et accueillir la tête sphérique d'un composant fémoral. La coupole comprend une première zone en calotte sphérique garnie d'un revêtement de titane poreux apte à être envahi par de l'os spongieux en croissance pour former ancrage secondaire. Une seconde zone lisse comporte des fentes suivant des méridiens de la coupole pour présenter une capacité de déformation élastique comparable à celle du cotyle dans la région des cornes.

Le certificat d'utilité FR 2 715 828 concerne une cupule destinée à être fixée sans ciment pour prothèse totale de la hanche. La cupule comporte trois calottes emboîtées dont celle extérieure présente des languettes en forme de portions de secteurs de calotte sphérique, définies par des fentes en arc de cercle qui s'étendent entre deux cercles parallèles au plan de l'ouverture de cette calotte, l'un étant situé près de ce plan et l'autre près du sommet de la calotte les extrémités des fentes près du sommet étant reliées, deux par deux, par des fentes partiellement annulaires coïncidant avec celui des cercles précités situés près du sommet, de sorte que toutes les languettes soient tenues par leur extrémité de grande dimension située près de l'ouverture et que l'ouverture de petite dimension, à savoir d'une portion sur deux de secteur sphérique, est déplaçable radialement, vers l'intérieur ou l'extérieur, seules les languettes portant sur leur face externe des picots d'ancrage.

Le EP-A-453 694 décrit également une cupule destinée à être fixée sans ciment à trois calottes sphériques.

Le brevet EP-A-0 353 171 décrit une cupule de prothèse du type comprenant:
■ une cupule de "soutien" destinée à être insérée dans l'os de l'articulation à renforcer ou à remplacer, et qui présente sur sa face convexe externe, d'une part, au moins une cheville expansible et, d'autre part, une pluralité de fentes d'expansion disposées sur des cercles méridiens passant par le pôle ouvertes sur l'équateur et une
■ une cupule de "frottement", en matière plastique destinée à s'insérer étroitement dans la cupule de soutien dont la face interne concave destinée à recevoir la tête de la tige de prothèse est hémisphérique.

Cependant lors de la marche sous les efforts d'appui, la cavité osseuse s'ovalise et se vrille ce qui crée des forces d'expulsion d'autant plus importantes que l'implant est de forme sensiblement sphérique, malgré les reliefs (aspérités, gorges ou crantages) dont est munie sa surface extérieure.

Un moyen efficace permettant à l'implant "d'accompagner" ces déformations osseuses, au lieu de s'y opposer, consiste à rendre l'armature au moins partiellement élastique pour reproduire les micro-mouvements osseux grâce à des ailettes déformables. Ce type d'implant existe et fonctionne relativement bien, associé avec un insert articulaire en matériau malléable pouvant suivre les mouvements de l'armature, comme le polyéthylène haute densité très utilisé pour ce genre d'application en frottement articulaire des prothèses totales de hanches.

Par contre lorsque l'insert articulaire est constitué par un matériau rigide comme l'alliage cobalt-chrome ou la céramique, l'armature dite élastique ne peut plus s'adapter avec les solutions existantes actuelles.

Il est un but de la présente invention de réaliser des implants cotyloïdiens améliorés surmontant les défauts des implants connus. Avec l'armature d'ancrage acétabulaire de la présente invention on assure l'ajustage serré de l'armature élastique par un effet ressort en utilisant un matériau de préférence métallique ayant une élasticité qui met l'os en pression.

L'armature d'ancrage acétabulaire selon la présente invention est définie dans la partie caractérisante de la revendication 1.

Pour que l'invention puisse être mieux comprise, référence est faite aux dessins accompagnants dans lesquels:
La figure 1 représente l'armature élastique d'ancrage acétabulaire selon la présente invention vue en perspective.
La figure 2 représente une vue de l'insert articulaire rigide selon l'invention utilisé avec l'armature d'ancrage acétabulaire de l'invention.
La figure 3 montre une vue en coupe de l'assemblage de l'insert articulaire rigide selon l'invention dans l'armature élastique d'ancrage acétabulaire de l'invention.
La figure 4 représente une autre vue en coupe de l'assemblage de l'insert articulaire rigide dans l'armature d'ancrage acétabulaire montrant certains détails de l'invention.

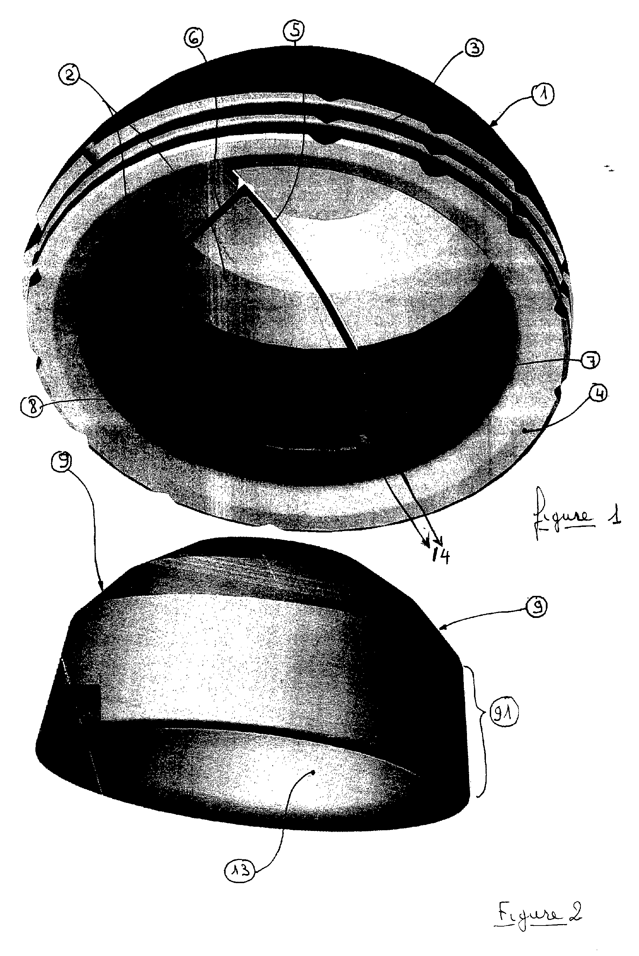

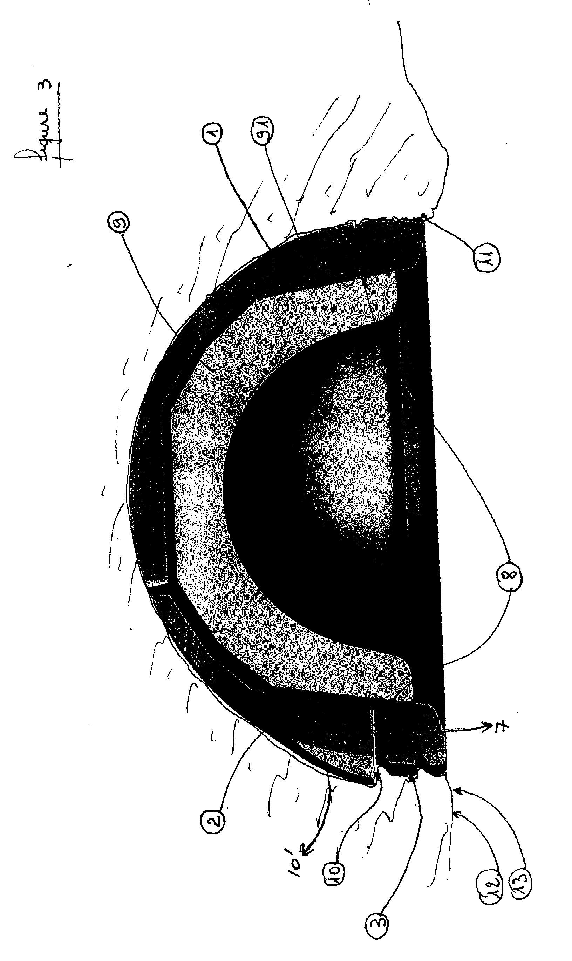

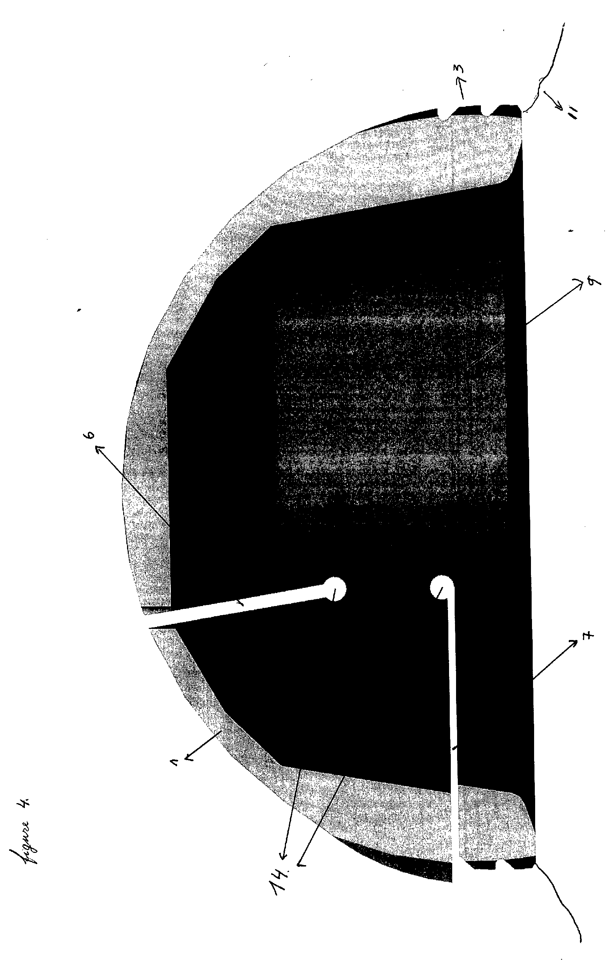

En se référant à la figure 1, l'armature élastique d'ancrage acétabulaire (1) comprend au moins une zone de déformation (2). A titre d'exemple, le mode de réalisation représenté dans la figure 1 comporte deux zones de déformation (2) et celles-ci sont en forme de pétales obtenues au moyen de deux fentes (5,6) sensiblement orthogonales partant du pôle de la géométrie extérieure de révolution (3) de l'armature, ce pôle ayant une forme globalement sphérique. En plus de ces fentes (5,6), l'armature comprend également une fente sensiblement équatoriale (7) (voir figs. 1 et 4) ces fentes (5,6 et 7) sont de préférence terminées par un orifice circulaire (14) de diamètre au moins égal à la largeur de la fente pour dissiper la contrainte due à la déformation élastique desdites pétales. La fente équatoriale (7) est sensiblement parallèle au plan d'ouverture (4) de l'armature d'ancrage là où les zones déformables (2) en forme de pétales dépassent la géométrie de révolution extérieure (3) de l'armature et lorsqu'on introduit l'armature dans une cavité osseuse acétabulaire, les zones déformables se rétractent par infraction en force de préférence avec ajustage serré de manière à créer un effet ressort. Grâce à l'élasticité du matériau de préférence métallique qui met en pression l'os, de préférence dans la zone des cornes inférieures (12 et 13), l'armature s'oppose, lors de la marche aux déformations d'ovalisation et de torsion de ladite cavité acétabulaire (11) qui tendent à expulser les armatures rigides d'ancrage.

La figure 2 représente un insert articulaire rigide (9) destiné à être introduit dans l'armature élastique d'ancrage acétabulaire (1). Cet insert articulaire rigide (9) comporte un cône extérieur de coincement (91) et une surface interne articulaire (13) sensiblement hémisphérique en matériau céramique, ou encore en alliage de cobalt-chrome, en titane et ses alliages, et en acier inoxydable. La condition à respecter est que cet insert articulaire rigide doit être de préférence identique au matériau de la tête articulaire de la prothèse totale de la hanche.

Similairement, la fente équatoriale (7) de l'armature d'ancrage acétabulaire est disposée à une distance par rapport au plan d'ouverture (4) de celui-ci de manière à constituer un anneau de coincement périphérique (8) destiné à enserrer à la manière d'une frette conique l'anneau de coincement (91) périphérique de manière à réaliser le blocage de l'insert articulaire rigide (9) dans l'armature (1).

La figure 3 montre l'assemblage de l'insert articulaire rigide (9) dans l'armature élastique d'ancrage acétabulaire (1). Cette figure met en évidence l'anneau de coincement périphérique (8) enserrant à la manière d'une frette conique l'insert articulaire rigide (9) et le contact du cône extérieur de coincement (91) de l'insert articulaire rigide (9) avec l'armature d'ancrage acétabulaire (1) sauf à l'aplomb des zones de déformation (2) en forme de pétales dont l'épaisseur est volontairement réduite afin de permettre leur rétraction sans venir en contact avec l'insert articulaire rigide (9) lors de la mise en place dans la cavité osseuse acétabulaire (11) par impaction en force. Selon un mode de réalisation préféré la zone de contact coinçant le cône extérieur de coincement (91) dans l'anneau de coincement périphérique (8) doit être supérieure à la demi-périphérie du cône d'assemblage, à cause de la fragilité relative des matières lorsque celles-ci sont en matériau céramique, cette zone de contact comprenant de préférence la totalité de la zone non déformable jusqu'aux fentes (5,6).

La rétraction desdites pétales (2) est obtenue par leur excentration extérieure (10) et intérieure (10') par rapport au volume de la géométrie extérieure de révolution (3) globalement sphérique et leur débordement par rapport au volume de la cavité osseuse acétabulaire (11). (voir fig. 3) Cette rétraction est rendue possible par l'espace ménagé entre l'armature (1) et les pétales (2), celle-ci correspondant de préférence à la possibilité de rétraction offerte par la largeur des fentes, tout en restant dans le domaine des déformations élastiques du matériau.

Selon un mode de réalisation préféré le matériau de l'armature l'élastique d'ancrage acétabulaire (1) est de préférence réalisé en un métal tel que le titane et ses alliages, un alliage de cobalt-chrome, de l'acier inoxydable ou en matière céramique telle que l'alumine ou la zircone ou des combinations de ceux-ci.

Grâce à l'assemblage d'insert articulaire rigide et de l'armature élastique d'ancrage acétabulaire selon l'invention, on peut donc assurer l'ajustage serré de l'armature élastique d'ancrage acétabulaire par effet ressort mettant l'os en pression.

L'invention a été décrite au moyen d'un mode de réalisation préféré et il doit être compris que des modifications peuvent être faites et ces modifications sont également couvertes dans les limites des revendications suivantes.

## Revendications

1. Armature d'ancrage acétabulaire (1) élastique pour pièce ou insert articulaire cotyloïdien (9) de hanche en matériau rigide à couple de frottement métal/métal ou matière céramique/matière céramique **caractérisée en ce qu'**elle présente au moins une zone déformable (2) dont l'épaisseur est volontairement réduite afin de permettre sa rétraction sans venir en contact avec l'insert ou pièce articulaire cotyloïdien rigide (9), cette zone déformable (2) dépassant de la géométrie de révolution extérieure (3) de ladite armature (1) et se rétractant lors de la mise en place dans la cavité osseuse acétabulaire (11) par impaction en force avec un ajustage serré de manière à créer un effet ressort grâce à l'élasticité du matériau qui la constitue mettant en pression l'os dans la zone des cornes inférieures (12 et 13) pour s'opposer lors de la marche aux déformations d'ovalisation et de torsion de la cavité acétabulaire (11).

2. Armature d'ancrage acétabulaire (1) élastique **caractérisée en ce que** la zone déformable (2) a une forme en pétales.

3. Armature d'ancrage acétabulaire (1) selon la revendication 1 **caractérisée en ce qu'**elle comprend au moins deux zones déformables (2).

4. Armature d'ancrage acétabulaire (1) selon la revendication 1 **caractérisée en ce que** la zone déformable (2) est en forme de pétale et est délimitée par des fentes à travers l'épaisseur de ladite armature comprenant au moins deux fentes (5,6) sensiblement orthogonales partant du pôle de la géométrie extérieure de révolution (3) globalement sphérique et au moins une fente équatoriale (7) sensiblement parallèle au plan d'ouverture (4) de l'armature d'ancrage.

5. Armature d'ancrage acétabulaire (1) selon la revendication 4 **caractérisée en ce que** ces fentes (5,6 et 7) sont terminées par un orifice circulaire (14) de diamètre au moins égal à la largeur de la fente pour dissiper les contraintes dues à la déformation élastique de la zone déformable (2).

6. Armature d'ancrage acétabulaire (1) selon la revendication 1 **caractérisée en ce que** ladite pièce articulaire cotyloïdienne (9) est maintenue dans l'armature d'ancrage acétabulaire (1) élastique par un contact intime, du cône extérieur de coincement conique (91) de l'insert articulaire (9) avec l'anneau de coincement périphérique (8) rigide de l'armature d'ancrage acétabulaire (1) mais pas à l'aplomb des zones de déformation (2).

7. Armature d'ancrage acétabulaire (1) 1 **caractérisée en ce que** la distance de la fente équatoriale (7) par rapport au plan d'ouverture (4) est déterminée de manière à constituer un anneau de coincement périphérique (8) destinée à insérer à la manière d'une frette conique l'insert articulaire rigide (9) à l'endroit de son cône extérieur de coincement (91).

8. Armature d'ancrage acétabulaire (1) **caractérisée en ce que** l'ajustage serré de cette armature élastique se fait par effet ressort grâce à l'élasticité du matériau dépassant la géométrie de révolution extérieure (3) de l'armature (1) mettant ainsi l'os en pression.

9. Armature d'ancrage acétabulaire (1) selon la revendication 8 **caractérisée en ce que** le matériau de l'armature est de préférence choisi parmi les métaux tels que le titane et ses alliages, l'acier inoxydable, les alliages de cobalt-chrome, ou des matières céramiques telles que l'alumine ou la zircone ou des combinaisons de celles-ci.

10. Armature d'ancrage acétabulaire (1) selon la revendication 1 **caractérisée en ce que** la rétraction des zones déformables, sous forme de pétales, est obtenue par leur excentration extérieure (10) et intérieure (10') par rapport au volume de la géométrie extérieure de révolution (3) globalement sphérique et leur débordement par rapport au volume de la cavité osseuse acétabulaire (11).

11. Armature d'ancrage acétabulaire (1) selon la revendication 10 **caractérisée en ce que** la rétraction des pétales est rendue possible par l'espace ménagé entre l'armature (1) et les pétales (2), celui-ci correspondant de préférence à la possibilité de rétraction offerte par la largeur des fentes, tout en restant dans le domaine des déformations élastiques du matériau.

12. Armature d'ancrage acétabulaire (1) selon l'une quelconque des revendications 1 à 11 **caractérisée en ce que** la zone de contact coinçant le cône extérieur de coincement (91) dans l'anneau de coincement périphérique (8) doit être supérieure à la demi-périphérie du cône d'assemblage, à cause de la fragilité relative des matières lorsque celles-ci sont en matériau céramique, cette zone de contact comprenant de préférence la totalité de la zone non déformable jusqu'aux fentes (5,6).
